# EUROPEAN PATENT SPECIFICATION

(11) **EP 1 989 290 B1**
(45) Date of publication and mention of the grant of the patent: **08.07.2015**
(21) Application number: 07752408.0
(22) Date of filing: 07.03.2007
(51) Int. Cl.: C12N 5/00, C12N 5/02, A61L 27/00

(54) **METHOD FOR ENDOTHELIAL CELL EXTRACTION FROM ADIPOSE TISSUES**
VERFAHREN ZUR EXTRAKTION VON ENDOTHELIALEN ZELLEN AUS FETTGEWEBE
PROCÉDÉ D'EXTRACTION DE CELLULES ENDOTHÉLIALES DE TISSUS ADIPEUX

(30) Priority: 07.03.2006 US 779454 P
(43) Date of publication of application: 12.11.2008
(73) Proprietor: Humacyte, Research Triangle Park NC 27709 (US)
(72) Inventor: NIKLASON, Laura, E., Greenwich, CT 06830 (US); LI, Yuling, Chapel Hill, NC 27514 (US)
(74) Representative: Tombling, Adrian George
(86) International application number: PCT/US2007/005706
(87) International publication number: WO 2007/103379

(56) References cited:
- US-A- 5 131 907
- US-A- 5 576 278
- US-A- 5 952 215
- US-A1- 2002 039 787
- US-A1- 2003 228 290
- US-A1- 2005 008 626
- US-A1- 2006 034 813
- ARTS C H P ET AL: "A novel method for isolating pure microvascular endothelial cells from subcutaneous fat tissue ideal for direct cell seeding" LABORATORY INVESTIGATION 2001 US, vol. 81, no. 10, 2001, pages 1461-1465, XP002515273 ISSN: 0023-6837
- WILLIAMS S K ET AL: "Adult human endothelial cell compatibility with prosthetic graft material" JOURNAL OF SURGICAL RESEARCH, ACADEMIC PRESS INC., SAN DIEGO, CA, US, vol. 38, no. 6, 1 June 1985 (1985-06-01), pages 618-629, XP023023942 ISSN: 0022-4804 [retrieved on 1985-06-01]
- TURNER N J ET AL: "A novel hyaluronan-based biomaterial (Hyaff-11<(>R)) as a scaffold for endothelial cells in tissue engineered vascular grafts" BIOMATERIALS, ELSEVIER SCIENCE PUBLISHERS BV., BARKING, GB, vol. 25, no. 28, 1 December 2004 (2004-12-01), pages 5955-5964, XP004512696 ISSN: 0142-9612
- ARTS C H P ET AL: "Application of a clinical grade CD34-mediated method for the enrichment of microvascular endothelial cells from fat tissue." CYTOTHERAPY 2004, vol. 6, no. 1, 2004, pages 30-42, XP009112270 ISSN: 1465-3249
- CURTI ET AL: "An Ultrastructural and Immunocytochemical Analysis of Human Endothelial Cell Adhesion on Coated Vascular Grafts" ANNALS OF VASCULAR SURGERY, QUALITY MEDICAL PUBLISHING, ST. LOUIS, MO, US, vol. 3, no. 4, 1 October 1989 (1989-10-01), pages 351-363, XP005940445 ISSN: 0890-5096
- JONULEIT H ET AL: "REGULATORY T-CELLS IN ANTITUMOR THERAPY ISOLATION AND FUNCTIONAL TESTING OF CD4+CD25+ REGULATORY T-CELLS" METHODS IN MOLECULAR MEDICINE, HUMANA PRESS, TOTOWA, NJ, US, vol. 109, 1 January 2005 (2005-01-01), pages 285-296, XP009065420 ISSN: 1543-1894
- HEWETT PETER W ET AL: "Immunomagnetic purification of human microvessel endothelial cells using Dynabeads coated with monoclonal antibodies to PECAM-1", EUROPEAN JOURNAL OF CELL BIOLOGY, WISSENSCHAFLICHE VERLAGSGESELLSCHAFT, STUTTGART, DE, vol. 62, no. 2, 1 January 1993 (1993-01-01), pages 451-454, XP009112370, ISSN: 0171-9335
- HEWETT P W ET AL: "ISOLATION AND CHARACTERIZATION OF MICROVESSEL ENDOTHELIAL CELLS FROM HUMAN MAMMARY ADIPOSE TISSUE", IN VITRO CELLULAR & DEVELOPMENTAL BIOLOGY -ANIMAL, SPRINGER, GERMANY, vol. 29A, no. 4, 1 April 1993 (1993-04-01) , pages 325-331, XP009008352, ISSN: 1071-2690

## Description

### TECHNICAL FIELD OF THE INVENTION

This invention is related to the area of cell culture and purification. In particular, it relates to endothelial cell culture and purification.

### BACKGROUND OF INVENTION

For many applications in cellular therapies and tissue engineering, it is necessary to obtain endothelial cells from patient tissue for transplantation back into that same patient. In order to minimize manufacturing costs, as well as to minimize any potential changes that occur to endothelial cells during culture, it is advantageous to isolate large numbers of endothelial cells quickly, within several hours. In order for such endothelial cells to be practically useful, it is often necessary to obtain large numbers of cells (*e.g., >* 1 million), and to have fairly high purity of isolated cells (*e.g*., > 90% endothelial identity). In addition, it is desirable to minimize time of isolation, which improves cellular viability and increases the ease of use.

Bypass surgery is a common treatment for coronary and peripheral vascular disease, which is the largest cause of mortality in both the USA and Europe (1,2). In 2004, 427,000 coronary bypass surgeries have been performed (2). The patency of autologous vein grafts is better than those with prosthetic grafts in bypass surgery; however, up to 30% of patients don't have suitable veins for bypass procedure. In these patients, small diameter prosthetic grafts are used, which results in comparatively high failure rate. The large difference in patency between prosthetic and autologous vein grafts could partially be attributed to a lack of endothelial cells (EC), which prevent thrombogenicity, on the luminal surface of prosthetic grafts (3, 4). Therefore, developing strategy to successfully seed EC on prosthetic vascular grafts would most likely improve the patency observed for these grafts.

EC seeding may be carried out in either single-stage or two-stage procedure (5). Two-stage seeding involving expansion of limited EC in vitro, which may take 4-5 weeks (6), therefore, is not appropriate for urgent patient care. In addition, expansion of EC in vitro requires GLP facility with high cost. Single-stage seeding is to isolate large number of EC and then immediately seed on prosthetic graft. Several investigators have tried to develop a single stage seeding procedure (5). Adipose tissue has been reported to contain abundant microvascular endothelial cells (MVEC) with easy accessibility (7, 8). Seeding vascular grafts with adipose-derived EC has enhanced patency in animal models (9-11), however, the results of clinical trials in human have been disappointing (12, 13). The reason could be that humans, unlike canines, lack self-endotheliazation capacity. In addition, contaminating non-endothelial cells isolated form adipose tissue contribute to intimal hyperplasia (14-17). Therefore, finding a quick and consistent method to isolated large number of EC with high purity is critical for the success of small diameter prosthetic graft implantation.

Generally speaking, two types of methods have been used to purify EC from different tissues. Positive selection involves application of magnetic beads conjugated with EC specific antibodies or molecules such as platelet endothelial cell adhesion molecule (PCAM/CD31), CD34, ve-cadherin (CD144), or Ulex europaeus agglutinin-1 (UEA-1) (18-20); negative depletion employs specific antibodies against non-endothelial cells to exclude cells such as fibroblasts or monocytes (21). Cells selected positively using CD34 Dynabeads or selected negatively using anti-fibroblast and monocytes antibodies were about 87% and 71% CD31-positive, respectively. However, EC recovery using CD34 beads is only about 24% (19). In addition, EC is usually isolated using crude collagenase, which shows substantial lot variation and needs validation each time with changing lots (22).

There is a continuing need in the art for faster and more successful endothelial cell recovery and seeding, for example, for *in vivo* uses.

### BRIEF SUMMARY OF THE INVENTION

A first embodiment of the invention provides a method of preparing endothelial cells from adipose tissue. Adipose tissue from a liposuction procedure is washed and cells are collected from the tissue. The cells are enzymatically treated with a purified preparation of collagenase. The preparation is depleted in pepsin, trypsin, and thermolysin. The preparation also includes purified dispase. The treated cells are sorted by contacting with magnetic beads comprising a first antibody specific for an antigen selected from a first group consisting of: CD31, CD34, CD144, and CD 146, or an antigen selected from a second group consisting of CD14, CD45, and F19. Cells which are bound to said magnetic beads are collected if the antibody is specific for an antigen in the first group, and cells which are not bound to said magnetic beads are collected if the antibody is specific for an antigen in the second group.

The method of the invention provides a population of endothelial cells isolated from adipose tissue. The population has the following properties:
>80 % of cells in the population are viable;
>80 % of cells in the population are endothelial cells;
>50 % of cells in the population adhere to a substrate
   within 24 hours of seeding on the substrate.

A prosthetic vascular graft comprising endothelial cells seeded onto its lumen can be produced. The endothelial cells adhere to the lumen at a density of >50,000 cells/cm².

The invention will be apparent to one of skill in the art upon reading the detailed description.

### BRIEF DESCRIPTION OF THE DRAWINGS

**Fig. 1****.** Cell yields with different enzymes for different incubation time
**Fig. 2****.** Cell viability with different enzymes for different incubation times.
**Fig. 3****.** Percentage of EC after digestion

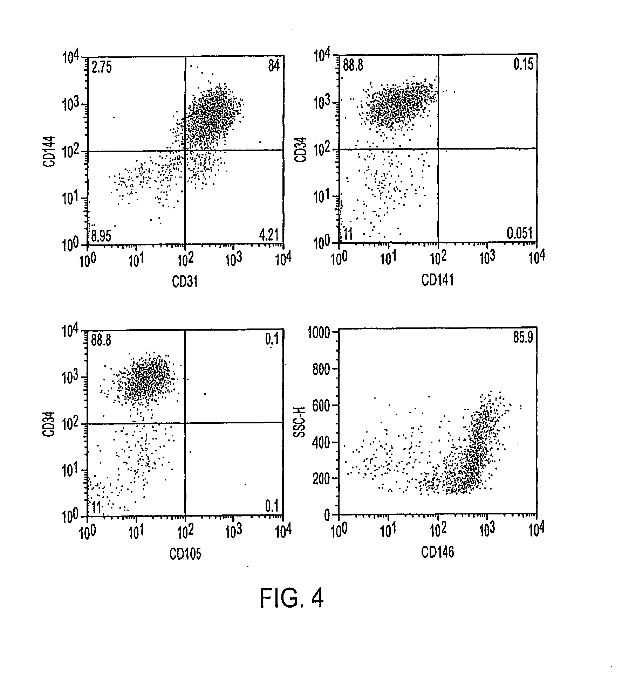

**Fig. 4****.** CD31 purified EC were characterized using FACS analysis.
**Fig. 5****.** Purified EC show cobble stone morphology in culture
**Fig.** 6A. Purified EC stain vWF positive in culture. Fig. 6B shows nuclear staining of cells.
**Fig.** 7. (Fig. 7A) Surface of non-treated engineered vascular graft, smooth collagenous surface, no endothelial cells present. (Fig. 7B) CD31 microbead purified EC attached to fibronectin coated, engineered vascular graft in 16hr. Graft is produced from decellularization of a tissue-engineered artery. These purified EC cells attach to fibronectin coated engineered vascular grafts very rapidly. About 50% of the surface area has been covered with purified EC in about 16hr. The approximate cell density in this example is 110,000 cells/cm2

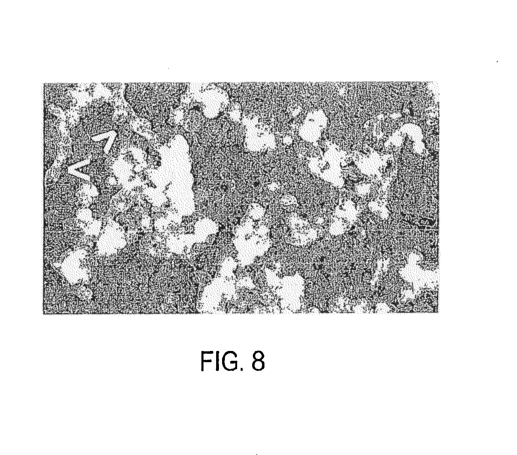

**Fig.** 8. Adipose-derived endothelial cells from fat, seeded for 16 hours onto engineered vascular graft. Dense cell seeding is evident. In addition, cell spreading onto the graft surface, indicative of firm adhesion, is noted by arrowheads.
**Fig.** 9A-9D. CD31+ cells were cultured in DMEM/10%FBS/MVGS on da y4. These cells were isolated from A: LB1; B: LB2; C: LB3; D: crude collagenase type I digestion with Fig. 9A: LB 1; Fig. 9B: crude collagenase type I; Fig. 9C: LB2; Fig. 9D: LB3

### DETAILED DESCRIPTION OF THE INVENTION

We describe a cell isolation method using purified collagenase types I and II, combined with purified dispase, to minimize enzyme variation and cellular damage during isolation. This purified enzyme formulation contributes to high viability and high plating efficiency of cells isolated by this method. We use CD31 microbeads to enrich fat-derived EC cells. As high as 84% of CD144 positive EC can be achieved. We found that EC purity after CD31 selection is related to the percentage of EC before selection. Purified EC express EC specific markers, such as CD31, CD34, CD144 and CD146 and they are negative in CD105, CD133, CD117 and CD141 expression or weak expressors thereof. These purified populations of EC from fat display very high viability and rapid attachment rate. These two characteristics of viability and plating, combined with high purity make this isolation method truly applicable to the clinic, in contrast to other methods that have been reported, which result in either low purity or low viability/plating efficiency. The EC cells which are isolated from fat many not have all the same properties as EC isolated from the vasculature.

Endothelial cells reside in all tissues of the body, in the form of microvascular and large vessel endothelium. In order to isolate cells from various tissues, it is typical to disaggregate the tissue, and then to select the endothelial cells from the balance of the cells that are also resident within the tissue. The present invention concerns a method of obtaining endothelial cells from tissues, which involves a disaggregation step, and an endothelial cell selection step. In some embodiments of the present invention, a centrifugation or other separation step may be utilized before or after disaggregation, to facilitate obtaining the endothelial cells.

For disaggregation of the tissue, several techniques may be used. In one embodiment, mechanical agitation and/or physical mincing may be employed to break up tissue architecture. Straining the tissue, or forcing through a sieve, may also disaggregate bulk tissues. Vigorous stirring may also be used. Alternatively (or in addition), proteases such as bacterial collagenase, elastase, or dispase may be utilized to break up the extracellular matrix that contains the tissue cells. Purified collagenase can be used, particularly those that are depleted in pepsin, trypsin, and/or thermolysin. Ion chelators such as EDTA may be utilized, which bind divalent cations that mediate cellular adhesion to matrix, thereby freeing cells from their surrounding proteins. All of these techniques may be performed at room temperature, or at temperatures higher than room temperature, such as 37° C, which may maximize the activity of various proteases. The pH of the incubating solution may be varied to increase the disaggregation of the tissue, with typical pH values ranging from 4.0 - 10.0. The times for application of these treatments can vary from 1 minute to as long as 24 hours, depending on the tissue density and strength of the extracellular matrix.

In some embodiments, a centrifugation step can be utilized after the disaggregation step as a means of collecting the cells. Centrifugation may occur in any type of standard buffer, or may occur in specialized centrifugation gradients solutions such as Ficoll gradients. Centrifugation steps may be particularly advantageous with tissues wherein the surrounding tissue has a different physical density than the endothelial cells. For example, endothelial isolation from adipose tissue or from bone marrow, both of which contain a high density of fat cells, can be improved by centrifugation. Centrifugation can separate low-density fat cells from higher density endothelial cells. However, in general, centrifugation alone is not sufficient for selective endothelial isolation from tissues. This is because other cells types, such as fibroblasts and perictyes, can have similar densities to endothelial cells. Hence, even if centrifugation is employed, a subsequent purification step is generally necessary to achieve high endothelial purity, for example of > 80, 85, 90, or 95 %. Tissue and cells can be washed in any cell-suitable buffer(s) including phosphate buffered saline, for example. Cell culture media may also be used. The washed tissue and/or cells can be decanted after settling or centrifugation to separate types of cells and cellular debris that migrates to different phases.

As an additional step in endothelial cell isolation, cellular selection is employed. Selection may be "positive," in that endothelial cell characteristics or markers are utilized to select the cells, or may be "negative," in that characteristics of other cell types within the tissue or centrifuged pellet may be utilized to exclude or remove those other cell types from the endothelial cells. Types of sorting procedures that are compatible with the present invention include magnetic bead isolation (MACS), fluorescence activated cell sorting (FACS), and elutriation. Examples of endothelial-specific markers that may be used for selection include the surface receptors for vascular endothelial growth factor (VEGF), vascular endothelial cadherin (VE-Cadherin), platelet endothelial adhesion molecule (PECAM, or CD-31), CD34 (Ligand for CD62 (L-selectin)), surface lectins (which are bound by UEA-1), von Willebrand factor, P-selectin, E-selectin, vascular endothelial cell adhesion molecule (VCAM-1), CD144 (Cadherin-5, VE-cadherin), CD146 (MCAM, MUC18, S-endo), and intercellular adhesion molecule (ICAM-1). Of these, those most advantageous for the present invention may include those that are expressed on the surface of non-activated endothelial cells, and would include CD-31, VE-cadherin, VEGF receptor, and lectins.. These lists are merely exemplary and not limiting.

Examples of negative selective markers would be those surface markers of other cell types within the tissue, and would be therefore somewhat tissue-specific. Many CD markers are compatible with present invention, especially those that recognize contaminating cell types in tissues, *e.g.,* fibroblasts. As a specific example, fibroblasts, pericytes and smooth muscle cells express the surface receptor for platelet-derived growth factor (PDGF receptor), and this marker can be used to select cells for exclusion or removal from the endothelial cell population. Particular antigens which can be targeted as a negative selection markers include CD 14, CD45, and F19.

If either FACS or magnetic bead cell sorting are used for cellular selection, then either one or some combination of the above types of positive or negative markers would be used to effect selection. In general, specific antibodies or other binding molecules for the cell-specific marker would be either bound to a fluorophore to allow FACS, or would be bound to magnetic beads to allow for cell separation by MACS. Either positive or negative selection may be utilized, or, in some embodiments, a combination of both positive and negative selection may be utilized. Alternatively, selection with several markers, positive and/or negative, may be utilized. Alternatively, elutriation can be used as a selection method; this method relies on specific size and density characteristics of the endothelial cells and other cells in the tissue. The specific range of endothelial sizes and densities, which may differ only slightly from sizes and densities of other cell types in the tissue, can be utilized to select the endothelial cells from the remaining cell types in the tissue.

In addition, it is envisioned that one particular method of selection does not preclude the use of additional methods. In other words, several methods of endothelial cell selection may be used either concurrently or in sequence, within the scope of the present invention. Certain steps can also be repeated to achieve better purification or yield.

Remarkably, the endothelial cell preparations produced by the present invention are highly and quickly adherent to appropriate substrates. Thus, we have observed that adherence to substrates occurs at a high rate and density and within a short period of time. Adherence can be assessed for example, at 12 hours, at 18 hours, at 24 hours, at 36 hours, at 48 hours, and/or at 72 hours post seeding. We have observed significant rates of adherence at times as short as these. The adherence can be evaluated on a vessel surface, such as a slide or culture vessel, or on a vascular graft. The quick adherence to substrates ("stickiness") and the high viability and the endothelial purity are among the signature properties of the populations produced by the present invention. Appropriate substrates are typically coated with extracellular matrix proteins. These may include collagen, fibronectin, and gelatin. Populations achieve at least 50 % adherence at 12 hours, at 18 hours, at 24 hours, at 36 hours, at 48 hours, and/or at 72 hours post seeding.

Populations of endothelial cells produced by the present invention are highly viable. Without being bound by any theory or mechanism, it is believed that prior art disaggregation methods were so harsh that viability and adhesiveness was adversely affected. The present populations are at least 50, 60, 70, 80, or even 90 percent viable.

Populations of endothelial cells produced by the present invention are highly pure. Using modern standards for assessing the identity of endothelial cells, i.e., using appropriate antigenic markers as described above, the present populations are at least about 50, 60, 70, 80, or even 90 percent endothelial cells. Such markers for endothelial cells include CD31⁺, CD34⁺, CD144⁺, CD146⁺, CD133⁻, CD45-, CD117-, and/or CD141⁻. Moreover, endothelial cells can be characterized by their ability to secrete tPA and prostacyclin in culture. All of these markers may not be equally well expressed in endothelial cell preparations. Again, without intending to be bound by any theory, it is postulated that prior art populations were unsuccessful for their intended purpose due to low ratios of real endothelial cells and/or low viability.

The combined results of high endothelial cell purity, high viability, and high adherence permits a liposuction sample to be taken from a patient to receive a vascular graft, to quickly process the sample so that the resulting cells can be used to populate the lumen of the vascular graft, and implant the vascular graft to the same patient within the same day or two or three days. Moreover, these properties permit the colonization by the endothelial cells of the lumen at a density of >50,000 cells/cm², >75,000 cells/cm², >85,000 cells/cm², >95,000 cells/cm², >105,000 cells/cm², or >110,000 cells/cm², Such high cell densities will increase the patency and decrease the failure of vascular grafts.

### Example 1

Subcutaneous fat tissue was obtained from liposuction or other surgical procedures. Fat tissue was subjected to mincing and then to bacterial collagenase to effect disaggregation. Disaggregated tissue was then centrifuged to separate adipose cells from other cell types (including endothelial cells), which reside in the centrifuged pellet. Re-suspension of the centrifuged pellet was followed by antibody-based selection for endothelial-specific markers. Fluorescence activated cell sorting for the endothelial surface molecule VE-cadherin was utilized. Under these conditions, each gram of fat tissue produced between 1.0 - 1.2 million cells in the centrifuged pellet after disaggregation. Cell sorting for VE-cadherin resulted in endothelial cell selection. We have found that the endothelial cell content in the centrifuged cell pellet is approximately 15-20 %. This translates to approximately 200,000 endothelial cells per gram of fat. The cellular viability of the endothelial cells, as assessed by 7AAD staining, is typically 85-90%. Hence, if 10 grams of subcutaneous fat are obtained from a given patient (corresponding to 2 teaspoons), approximately 2 million living endothelial cells are obtained, which is a sufficient number to line the inside of a vascular graft, such as might be used for bypass surgery. The purity of the isolated endothelial cells, as assessed by repeat fluorescence activated cell sorting, was approximately 90%.

### Example 2

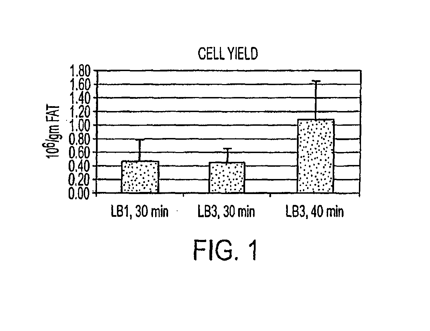

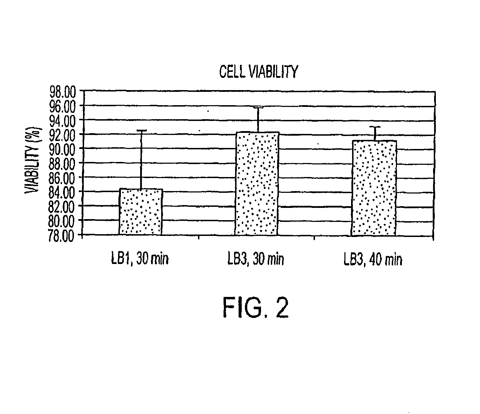

We tested Liberase Blendzymes (Roche Diagnostics, Indianapolis, Indiana) for digesting liposuction tissue and releasing endothelial cells (EC). Liberase Blendzymes consist of purified collagenase and other proteases and different batches have the same enzyme activity. Therefore, variation resulting from enzyme lots can be avoided. Cell yield (number of cells/gm fat) and cell viability (measured using 7-AAD by FACS) are shown in Fig. 1 and Fig. 2. Average cell yields are similar between LB 1 and LB3 with the same incubation time (30min), about 5x10⁵ cells per gram of fat. Longer incubation with LB3 (40min) almost doubles the cell yield (1x10⁶) with comparable cell viability.

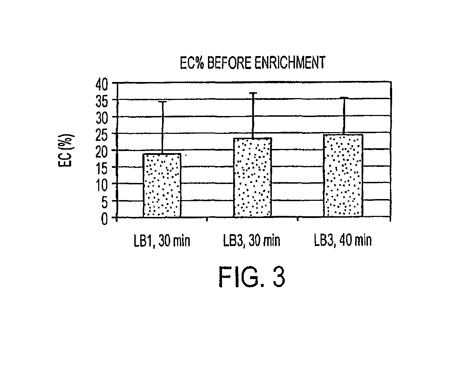

Percentage of EC as a fraction of the total non-adipocyte cells released from fat tissue is similar using different Liberase Blendzyme enzymes and digestion times, as shown. (Fig. 3.) These cells are not purified by any means after enzymatic digestion, but merely characterized by FACS sorting for endothelial-specific markers. These data show that, following enzymatic digestion, the non-adipocyte cell population is mixed, with a fraction of endothelial cells that are mixed with other cell types. Without purification of this mixed cell population that results from enzymatic digestion, this mixed population is not optimal for seeding onto a vascular graft, since contaminating fibroblasts and other cell types could contribute to intimal hyperplasia and graft failure.

Mixed cell populations derived after enzymatic digestion are purified using positive selection with CD31 microbeads. Purified EC express CD31, CD34, CD144, and CD146; and they are CD105 and CD141 negative (Fig..4). The endothelial purity of this population is greater than 80%, as assessed by CD144 (VE-cadherin) expression, a marker that is highly specific for endothelium.

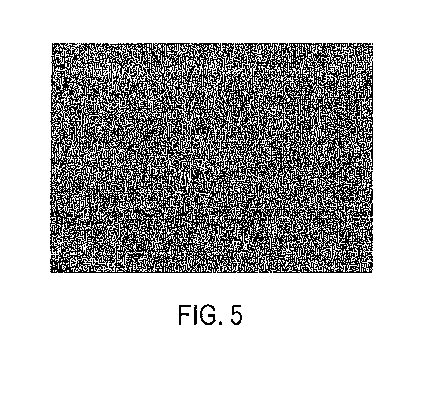

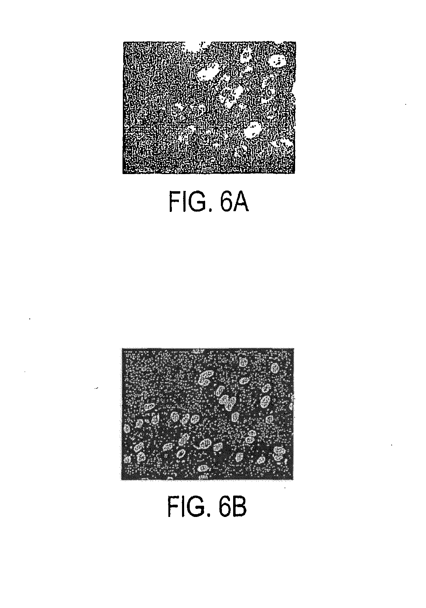

Purified EC grow in culture, show typical EC cobble stone morphology (Fig. 5), and stain von Willebrand factor-positive with immnocytochemistry (Fig. 6). Von Willebrand factor (vWF) is a marker for highly differentiated endothelium, showing the high functionality of the EC isolated with this technique.

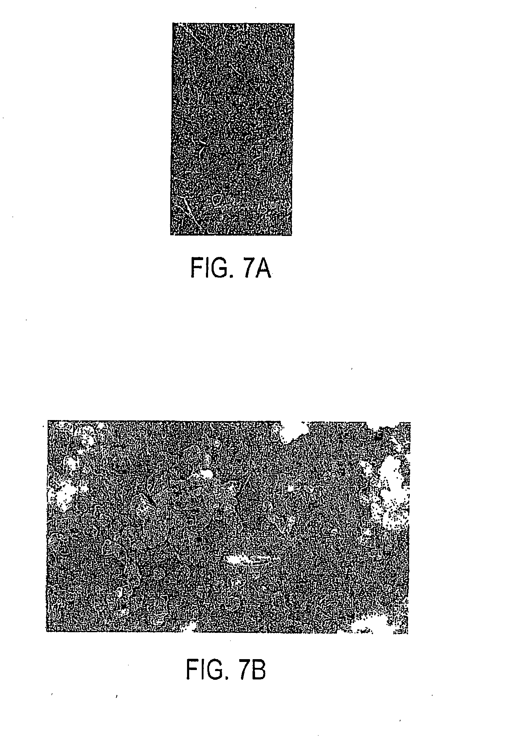

The purified EC cells attach to fibronectin-coated, engineered, vascular grafts in about 10 hr. The graft is produced from decellularization of a tissue-engineered artery. The decellularized graft luminal surface, without endothelium, is smooth andproteinaceous (Fig. 7A). The purified EC cells attach to fibronectin-coated engineered vascular grafts very rapidly. About 50 % of the surface area has been covered with purified EC in about 16 hr. The approximate cell density in this example is 110,000 cells/cm². (Fig. 7B).

Positive selection using anti-CD31 microbeads has been tested for enrichment of EC. Percentage of cells expressing CD144, an EC specific marker, was used to measure the purity of EC. EC purity after CD31 enrichment is directly related to the percentage of EC before enrichment (Table 1). The highest EC purity observed with this method is about 87%. This reflects the significant variation among individuals regarding EC percentage before purification.

**Table 1. EC purity after enrichment is dependent on EC% prior to enrichment**

| Enrichment methods | EC% before enrichment | EC% after enrichment |
|---|---|---|
| CD31, autoMACS | 2.91 | 17.2 |
| | 9.87 | 56.2 |
| | 10 | 40 |
| | 12 | 58.8 |
| | 13.3 | 41.2 |
| | 15.9 | 52.4 |
| | 21.4 | 84 |
| | 28.5 | 86.7 |
| | 42.1 | 73.3 |

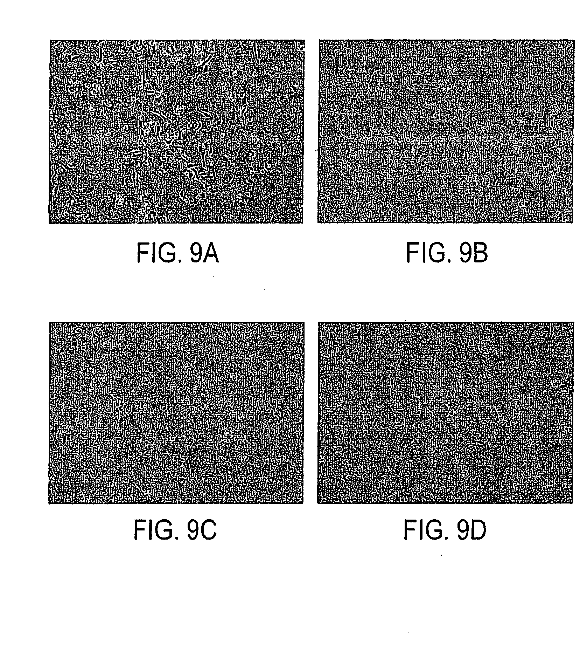

Cells released from LB1 digestion had the highest plating efficiency after culture in EBM-A media for 10 hrs (Table 2, **FIG.** 9A-9D). The plating efficiency is at least 58% from LB1 digestion. This is significantly higher than that observed with LB2 or LB3. Importantly, the plating efficiency is much higher than with crude collagenase that is utilized by others. One key aspect of the invention is the rapid plating (in this case, in 10 hours or less) of a high fraction of purified EC from adipose tissue. The underlying reason that cells released using LB 1 and then purified have such a high plating efficiency could be that dispase (in LB1) is a more gentle enzyme than thermolysin (in LB3), and cells digested with LB1 may as a result retain receptors necessary for cell attachment. In addition, non-purified collagenase may damage cells also, and result in a low plating efficiency as compared to highly purified forms of enzyme.

**Table 2. Plating efficiency 10 hr post culture in EBM-A**

| LB1 | LB2 | LB3 | Col I |
|---|---|---|---|
| 58.5 | <5% | <5% | <5% |
| 65.4 | <5% | <5% | <5% |
| 83.6 | | | |

**Table 3: EC recovery after CD31 microbead purification**

| | Starting cells (10⁶) | EC% before purification | CD31⁺ collection (10⁶) | EC% after purification | EC recovery¹ (%) |
|---|---|---|---|---|---|
| | 15.5 | 2.91 | 2.2 | 17.2 | 0.84 |
| | 22.4 | 9.87 | 2.7 | 56.2 | 0.69 |
| | 43 | 13.3 | 8.1 | 41.2 | 0.58 |
| | 49 | 10 | 4.8 | 40 | 0.39 |
| | 51 | 12 | 6.7 | 58.8 | 0.64 |
| | 23.4 | 21.4 | 4.44 | 84 | 0.74 |
| | 48.6 | 15.9 | 9.3 | 52.4 | 0.63 |
| | 55 | 28.5 | 13.44 | 86.9 | 0.75 |
| | 15 | 42.1 | 3.36 | 73.3 | 0.39 |
| | 50 | 28.5 | 13.44 | 86.7 | 0.82 |
| Mean | | | | | 0.65 |
| STDEV | | | | | 0.16 |

| | | | | | |
|---|---|---|---|---|---|
| ¹ EC recovery (%) = (starting cell number x EC% before purification)/(CD31 positive collection x EC% after purification) x 100 | | | | | |

### Example 3- Materials and Methods

### 1. Materials

### 1.1. Tissue

Adipose tissue samples obtained from liposuction aspirates or dissected subcutaneous fat tissue

### 1.2. Reagents

1.2.1. Phosphate Buffer Saline without calcium and magnesium (PBS (1X)) or Hanks'
   Balanced Salt Buffer without calcium and magnesium (HBSS (1x)) (Gibco)
1.2.2. M199 media (Gibco)
1.2.3. EBM-2 media (Clonetics)
**1.2.4.)** RPMI (Gibco)
**1.2.5.** Bovine Serum Albumin (BSA) (Miltenyi Biotech)
**1.2.6.** Liberase Blendzyme 1 and Liberate Blendzyme 3 (Roche)
**1.2.7.** Collagenase type I (Sigma)
**1.2.8.** L-glutamine (Invitrogen)
**1.2.9.** Hydrocortisone (Sigma)
**1.2.10.** Dibutyryl cyclic AMP (Sigma)
**1.2.11.** Penicillin-Streptomycin Solution (100x Sigmal)
**1.2.13.** Trypsin-EDTA (0.25 mg/ml) (Invitrogen)
**1.2.14.** Ethylenediamine-tetraacetic acid disodium salt (EDTA) (Sigma)
**1.2.15.** Fetal Bovine Serum (FBS) qualified, heat inactivated (US) (Gibco
**1.2.16.** CD31 MicroBeads (Miltenyi Biotech)
**1.2.17.** FcR blocking reagent (Miltenyi Biotech)
**1.2.18.** CD 31-Fitc (BD)
**1.2.19.** CD 45-APC (BD)
**1.2.20.** CD105 Fitc (Chemicon)
**1.2.21.** CD117 APC (BD)
**1.2.22.** CD133 PE (BD)
**1.2.23.** Thrombomodulin (CD 141-PE, BD)
**1.2.24.** Ve-cadherin-PE (CD 144-PE, eBiosciences)
**1.2.25.** CD146-PE (BD)
**1.2.26.** Uea-1-fitc (Biomeda)
**1.2.27.** 7-AAD (BD)
**1.2.28.** vWF (Von Willebrand Factor) antibody (Dako)
**1.2.29. 4%** paraformaldehyde (USB)
**1.2.30.** Triton X-100 (Sigma)
**1.2.31.** Tween-20 (Sigma)
**1.2.32.** 4',6-diamidino-2-phenylindole, dihydrochloride (DAPI) (Molecular Probes)

### 1.3. Supplies

**1.3.1.** 500 ml plastic centrifugation bottles (Corning)
**1.3.2. 0.2** µm filter units (Nalgene)
**1.3.3.** 50 ml conical tubes (Corning)
**1.3.4. 15** conical tubes (Corning)
**1.3.5.** 5 ml polystyrene tubes (BD)

### 1.4 Equipment

**1.4.1.** AutoMACS Separator is benchtop automated magnetic cell sorters (Miltenyi Biotech)
**1.4.2.** BD FACSCalibur system (BD)
**1.4.3.** Titer Plate Shaker (Lab-Line Instruments)
**1.4.3.** Centrifuge (Beckman)
**1.4.4.** Biosafety Hood
**1.4.5.** CO₂ Incubator (NUAIR)
**1.4.6.** Inverted microscope -Nikon Eclipse TS100 with Epi-Fluorescence Attachment (Mercury Lamp Illuminator model name: C-SHG) (Nikon Instruments Incorporation, Melville, NY) and equipped with a camera photometric cool-snap (Nikon)

### 1.5. Media stock solution

All the media solutions are filtered through a 0.2 µm filter unit and Frozen down in 50ml tube in -20°C..

### 1.5.1. EBM-A medium

EBM-2 base medium (CLOTECH)
20% FBS
L-glutamine (0.292 mg/ml)
Hydrocortisone (1 µg/ml)
Di cAMP (0.25 mg/ml)
1% Penicillin-Streptomycin Antibiotic solution

### 1.5.2. DMEM BASED medium

DMEM (Invitrogen)
10% FBS
1x MVGS (Cascade Biologics)
Penicillin-Streptomycin

### 2. Methods

After transportation to the laboratory, the liposuction sample is immediately processed to isolate MVEC. If sample can't be processed immediately, store it at room temperature and process it in 24 hrs. Before performing the experiment, warm up the water bath to 37°C.
All the following procedures are performed in Biosafety Hoods
2.1. Isolation
2.1. 1. Warm up buffer 500 or more of PBS or HBSS with 0.1% glucose. Line the surface of the biosafety hood with a disposable bench protector.
2.1.2. Prepare digestion solution (0.75 u/ml Liberase Blendzyme 1 (LB1) or Liberase Blendzyme 3(LB3) or 4mg/ml collagenase type I in PBS or HBSS with 4mg/ml BSA and 0.1% glucose), and warm it in the 37°C waterbath.
**2.1.3.** Warm M199 and EC media in the 37°C waterbath
**2.1.4.** Prepare RPMI/1% FBS/2mM EDTA and keep it at 4°C
**2.1.5.** To maintain optimal sterile conditions, open the surgical container used for liposuction procedure under the biosafety hood.
   Dispense 200 ml of adipose tissue in 500 ml centrifuge bottles (Coming). Add an equal volume of warm PBS or HBSS. Agitate to wash the tissue and then allow phase separation for 3-5 min. Aspirate the infranatant solution (lower liquid phase).
   The wash is repeated several times until a clear infranatant solution is obtained (usually 3-4 times).
**2.1.6.** Centrifuge cells at 1500-2000 rpm for 5-15 min after last wash. Aspirate the infranatant solution. Measure adipose tissue and add about 1ml of warm LB1 or LB3, or collagenase type I solution per 1 g of fat. Tight the lid and place them on a shaker in a 37°C incubator with gentle shaking for 30-40min. Mix tissue manually every 10 min.
**2.1.7..**Add 150 ml of warm M199 media and centrifuge cells at 1200rpm for 5 min.
**2.1.8-**After spinning, EC as well as other cells will form a pellet at the bottom of the bottle or tube (this will usually include a layer of dark red cells). Carefully remove the top layer of oil and fat, the primary adipocytes (a yellow layer of floating cells), and the underlying layer of digestion solution. Leave behind a small volume of collagenase solution above the pellet so that the cells are not disturbed.
2.1.9. Suspend the cells in 10 ml of warm M199 media and filter cells with a 70um cell strainer. Centrifuge the cells at 1200 rpm in an appropriate centrifuge for 5 minutes at room temperature.
**2.1.10.** Aspirate the remaining media. When aspirating, the tip of the pipette should aspirate from the top so that the oil is removed as thoroughly as possible. The cell pellet should be at the bottom of the tubes.
**2.1.11.** Resuspend the cells with 10 ml of cold RPMI/1% FBS/2mM EDTA medium in each tube. Pool the cells in one 50 ml conical tube. Filter cells through a 70um stainer.
**2.1.12.** Remove 150 ul cell suspension and count cells using Sysmex.

### 2.2. EC enrichment with CD31 microbeads

**2.2.1.** Transfer 25 x 10⁶ -50 x 10⁶ cells to a 15 ml conical tube and centrifuge cells at 1200 rpm for 5 minutes at room temperature.
**2.2.2.** Aspirate off the supernatant and suspend the cells to a maximum concentration of 10 x 10⁶ cells per 60 ul of RPMI/1% FBS/2mM EDTA medium. Add 20ul of FcR blocking reagent per 10 x 10⁶ cells. Mix briefly, and then add 20 ul of CD31-MicroBeads per 10 x 10⁶ cells. Incubate cells for 15 min at 4°C.
**2.2.3.** After incubation, rinse cells with RPMI/1% FBS/2mM EDTA medium and centrifuge cells at 300xg for 10 min.
**2.2.4.** Suspend cell pellet in 2 ml of RPMI/1% FBS/2mM EDTA medium.
**2.2.5.** Load cells on autoMACS and separated using program POSSELD.
**2.2.6.** Collect CD31 positive and negative cells.
**2.2.7.** Transfer 150 ul of either CD31 positve or negative cells to a micro centrifuge tube and count cells using Sysmex.
**2.2.8.** Characterize cells before and after CD31 separation using using Fluorescence Activated Cell Sorting (FACS). Viability is measured using FACS by staining cells with 7-AAD.

### 2.3 Plating efficiency:

2.3.1. Seed CD31 purified EC with a density of 2-5x 10⁵/cm² in ECM coated, such as fibronectin, geletin, collagen I, with EC media such as EBM-A or MEM/10%FBS/MVGS and cultured in 5% CO2, 37C incubator.
2.3.2. Next day, shake plate gently and collect media containing unattached cells in a tube. Rinse cells with PBS and collect cells in the same tube. Add fresh media to the plate and place back to incubator.
2.3.3. Centrifuge collected media 1500rpm for 5 min.
**2.3.4.** Aspirate media and suspend cells in 200 to 500 ul PBS. Vigorously pipet cells and count cells using Sysmem.
**2.3.5.** Plating efficiency = (number of total seeded cells- number of floating cells)/total seeded cells).

### 2.4. Cell characterization

Cells before and after CD31 microbeads separation as well as cultured for 48 hr are collected and characterized by FACS analysis for CD31, CD34, CD45, CD141 and CD144, CD146 expression. Some cells from 96 well plates were fixed with 4% paraformaldehyde and stained with vWF, eNOS or CD31.

### 2.5. Seed EC on prosthetic grafts

A 0.5 x 0.5 cm piece of Humacyte™ engineered grafts were coated with human fibronectin (100ug/ml) in 6-well plate for 1-8hr at 37°C. CD31 microbead selected EC (1 x 10⁶-5 x 10⁶) were then added in the well and incubated for 10-16 hr in the incubator. The grafts were fixed in formalin and scanning electron microscopy (SEM) was performed on these grafts.

### References:

1. American Heart Association - Heart Disease and Stroke Statistics. 2006 Update. http://www.americanheart.org/downloadable/heart/1166711577754HS _StatsInsideText.pdf
2. Pomposelli FB Jr, Arora S, Gibbons GW, Frykberg R, Smakowski P, Campbell DR, Freeman DV, LoGerfo FW. Lower extremity arterial reconstruction in the very elderly: successful outcome preserves not only the limb but also residential status and ambulatory function. J Vasc Surg. 1998; 28(2):215-25.
3. Veith FJ, Moss CM, Sprayregen S, Montefusco C. Preoperative saphenous venography in arterial reconstructive surgery of the lower extremity. Surgery. 1979; 85(3):253-6.
4. Veith FJ, Gupta SK, Ascer E, White-Flores S, Samson RH, Scher LA, Towne JB, Bernhard VM, Bonier P, Flinn WR, et al. Six-year prospective multicenter randomized comparison of autologous saphenous vein and expanded polytetrafluoroethylene grafts in infrainguinal arterial reconstructions. J Vasc Surg. 1986; 3(1):104-14.
5. Tiwari A, Salacinski HJ, Hamilton G, Seifalian AM. Tissue engineering of vascular bypass grafts: role of endothelial cell extraction. Eur J Vasc Endovasc Surg. 2001; 21(3):193-201. Review.
6. Zilla P, Fasol R, Dudeck U, Siedler S, Preiss P, Fischlein T, Muller-Glauser W, Baitella G, Sanan D, Odell J, et al. In situ cannulation, microgrid follow-up and low-density plating provide first passage endothelial cell masscultures for in vitro lining. J Vasc Surg. 1990; 12(2):180-9.
7. Jarrell BE, Williams SK, Stokes G, Hubbard FA, Carabasi RA, Koolpe E, Greener D, Pratt K, Moritz MJ, Radomski J, et al. Use of freshly isolated capillary endothelial cells for the immediate establishment of a monolayer on a vascular graft at surgery. Surgery. 1986; 100(2):392-9.
8. Williams SK, Jarrell BE, Rose DG, Pontell J, Kapelan BA, Park PK, Carter TL. Human microvessel endothelial cell isolation and vascular graft sodding in the operating room. Ann Vasc Surg. 1989 Apr; 3(2):146-52.
9. Pasic M, Muller-Glauser W, von Segesser L, Odermatt B, Lachat M, Turina M. Endothelial cell seeding improves patency of synthetic vascular grafts: manual versus automatized method. Eur J Cardiothorac Surg. 1996; 10(5):372-9.
10. Williams SK, Rose DG, Jarrell BE. Microvascular endothelial cell sodding of ePTFE vascular grafts: improved patency and stability of the cellular lining. J Biomed Mater Res. 1994 Feb; 28(2):203-12.
11. Williams SK, Jarrell BE, Kleinert LB. Endothelial cell transplantation onto polymeric arteriovenous grafts evaluated using a canine model. J Invest Surg. 1994 Nov-Dec; 7(6):503-17.
12. Park PK, Jarrell BE, Williams SK, Carter TL, Rose DG, Martinez-Hernandez A, Carabasi RA 3rd. Thrombus-free, human endothelial surface in the midregiori of a Dacron vascular graft in the splanchnic venous circuit--observations after nine months of implantation. J Vasc Surg. 1990; 11(3):468-75.
13. Meerbaum SO. Sharp WV, Schmidt SP. Lower extremity revascularization with polytetrafluoroethylene grafts seeded with microvscular endothelial cells. In: Zilla, P, Fasol, R. Callow, A. editors. Applied Cardiovascular Biology 1990-91. International Society For Applied Cardiovascular Biology. 2nd ed. Basel: Karger, 1992, pp107-119 et al.
14. Sharp WV, Schmidt SP, Meerbaum SO, Pippert TR. Derivation of human microvascular endothelial cells for prosthetic vascular graft seeding. Ann Vasc Surg. 1989; 3(2):104-7.
15. Sterpetti AV, Hunter WJ, Schultz RD, Sugimoto JT, Blair EA, Hacker K, Chasan P, Valentine J. Seeding with endothelial cells derived from the microvessels of the omentum and from the jugular vein: a comparative study. J Vasc Surg. 1988; 7(5):677-84.
16. Arts CH, Hedeman Joosten PP, Blankensteijn JD, Staal FJ, Ng PY, Heijnen-Snyder GJ, Sixma JJ, Verhagen HJ, de Groot PG, Eikelboom BC. Contaminants from the transplant contribute to intimal hyperplasia associated with microvascular endothelial cell seeding. Eur J Vasc Endovasc Surg. 2002; 23(1):29-38.
17. Arts CH, Blankensteijn JD, Heijnen-Snyder GJ, Verhagen HJ, Hedeman Joosten PP, Sixma JJ, Eikelboom BC, de Groot PG. Reduction of non-endothelial cell contamination of microvascular endothelial cell seeded grafts decreases thrombogenicity and intimal hyperplasia. Eur J Vasc Endovasc Surg. 2002; 23(5):404-12.
18. Hewett PW, Murray JC. Immunomagnetic purification of human microvessel endothelial cells using Dynabeads coated with monoclonal antibodies to PECAM-1. Eur J Cell Biol. 1993; 62(2):451-4.
19. Arts CH, de Groot P, Heijnen-Snyder GJ, Blankensteijn JD, Eikelboom BC, Slaper-Cortenbach IC. Application of a clinical grade CD34-mediated method for the enrichment of microvascular endothelial cells from fat tissue. Cytotherapy. 2004; 6(1):30-42.
20. Miebach S, Grau S, Hummel V, Rieckmann P, Tonn JC, Goldbrunner RH. Isolation and culture of microvascular endothelial cells from gliomas of different WHO grades. J Neurooncol. 2006; 76(1):39-48.
21. Arts CH, Heijnen-Snyder GJ, Joosten PP, Verhagen HJ, Eikelboom BC, Sixma JJ, de Groot PG. A novel method for isolating pure microvascular endothelial cells from subcutaneous fat tissue ideal for direct cell seeding. Lab Invest. 2001; 81(10):1461-5.
22. Williams SK, McKenney S, Jarrell BE. Collagenase lot selection and purification for adipose tissue digestion. Cell Transplant. 1995; 4(3):281-9

## Claims

1. A method of preparing endothelial cells from adipose tissue, comprising:
washing adipose tissue obtained from a liposuction procedure patient;
collecting cells from the washed adipose tissue;
enzymatically treating the cells with a purified preparation of collagenase and dispase, wherein said preparation is depleted in pepsin, trypsin, and thermolysin;
sorting the treated cells by contacting with magnetic beads comprising a positive selective antibody specific for an antigen selected from a first group consisting of: CD31, CD34, CD144, and CD 146, or a negative selective antibody specific for an antigen selected from a group consisting of CD14, CD45, and F19; and
collecting cells which are bound to said magnetic beads if the positive selective antibody is used or collecting cells which are not bound to said magnetic beads if the negative selective antibody is used.

2. The method of claim 1 wherein the collected cells are seeded onto a vascular graft within 3 days of liposuction procedure, preferably within 2 days of liposuction procedure and more preferably within 1 days of liposuction procedure.

3. The method of claim 2 wherein the vascular graft is for implantation into the liposuction procedure patient.

4. The method of claim 1 wherein magnetic beads comprising more than one antibody are contacted with the treated cells.

5. The method of claim 1 wherein the collagenase is from *Clostridium histolyticum.*

6. The method of claim 1 wherein the dispase is from *Bacillus polymixa.*

7. The method of claim 1 further comprising seeding the collected cells onto a vascular graft.

## Patentansprüche

1. Verfahren zum Zubereiten endothelialer Zellen aus adipösem Gewebe, aufweisend:
Waschen von adipösem Gewebe, das aus einer Fettabsaugung eines Patienten erhalten wurde;
Sammeln von Zellen aus dem gewaschenen adipösen Gewebe;
enzymatisches Behandeln der Zellen mit einem aufbereiteten Präparat auch Kollagenase und Dispase, wobei das Präparat hinsichtlich Pepsin, Trypsin und Thermolysin abgereichert ist;
Sortieren der behandelten Zellen durch in Kontakt bringen mit Magnetpartikel, die einen positiven selektiven Antikörper, der spezifisch für ein Antigen ist, das aus einer ersten Gruppe ausgewählt ist bestehend aus: CD31, CD34, CD144 und CD146, oder einen negativen selektiven Antikörper, der spezifisch für ein Antigen ist, das aus einer Gruppe ausgewählt ist bestehend aus CD14, CD45 und F19, aufweisen; und
Sammeln derjenigen Zellen, die an die Magnetpartikel gebunden sind, falls der positive selektive Antikörper verwendet wurde, oder Sammeln derjenigen Zellen, die nicht an die Magnetpartikel gebunden sind, falls der negative selektive Antikörper verwendet wurde.

2. Verfahren nach Anspruch 1, wobei die gesammelten Zellen auf ein vaskuläres Gefäßimplantat innerhalb von drei Tagen nach der Fettabsaugung, vorzugsweise innerhalb von zwei Tagen nach der Fettabsaugung und noch bevorzugter innerhalb von einem Tag nach der Fettabsaugung eingepflanzt werden.

3. Verfahren nach Anspruch 2, wobei das vaskuläre Gefäßimplantat zur Implantierung in einen Patienten dient, der einer Fettabsaugung unterzogen wurde.

4. Verfahren nach Anspruch 1, wobei die Magnetpartikel, die mehr als einen Antikörper aufweisen, in Kontakt mit den behandelten Zellen gebracht werden.

5. Verfahren nach Anspruch 1, wobei die Kollaginase aus *Clostridium histolyticum* ist.

6. Verfahren nach Anspruch 1, wobei die Dispase aus *Bacillus polymixa* ist.

7. Verfahren nach Anspruch 1, des Weiteren aufweisend das Einpflanzen der gesammelten Zellen auf ein vaskuläres Gefäßimplantat.

## Revendications

1. Procédé permettant de préparer des cellules endothéliales à partir de tissus adipeux comprenant les étapes consistant à :
laver un tissu adipeux obtenu à l'issue d'un procédé de liposucions d'un patient,
recueillir des cellules du tissu adipeux lavé,
effectuer un traitement enzymatique des cellules avec une préparation purifiée de collagénase et de dispase, cette préparation étant appauvrie en pepsine, trypsine et thermolysine,
trier les cellules traitées en les mettant en contact avec des billes magnétiques comprenant un anticorps sélectif positif spécifique à un antigène choisi dans un premier groupe formé par : CD31, CD34, CD 144, et
CD 146, ou un anticorps sélectif négatif spécifique à un antigène choisi dans le groupe formé par CD14, CD45 et F19, et
recueillir des cellules qui sont liées aux billes magnétiques si l'anticorps sélectif positif est utilisé ou recueillir les cellules qui ne sont pas liées aux billes magnétiques si l'anticorps sélectif négatif est utilisé.

2. Procédé conforme à la revendication 1, selon lequel les cellules recueillies sont ensemencées sur un greffon vasculaire dans les trois jours suivant la liposuccion, de préférence dans les deux jours suivant la liposuccion et de façon plus préférentielle dans le jour suivant la liposuccion.

3. Procédé conforme à la revendication 2, selon lequel le greffon vasculaire est destiné à être implantée chez le patient ayant subi la liposuccion.

4. Procédé conforme à la revendication 1, selon lequel des billes magnétiques renfermant plus d'un anticorps sont mises en contact avec les cellules traitées.

5. Procédé conforme à la revendication 1, selon lequel la collagénase est dérivée de *Clostridium histolyticum.*

6. Procédé conforme à la revendication 1, selon lequel la dispase est dérivée de *Bacillus polymixa.*

7. Procédé conforme à la revendication 1, comprenant en outre, une étape consistant à ensemencer les cellules recueillies sur un greffon vasculaire.
